(19) Europäisches Patentamt
European Patent Office
Office européen des brevets

(11) Veröffentlichungsnummer: **0 148 401**
**B1**

(12) **EUROPÄISCHE PATENTSCHRIFT**

(45) Veröffentlichungstag der Patentschrift: **01.04.87**

(51) Int. Cl.⁴: **C 07 C 125/06**, C 07 C 127/17, C 08 K 5/18, C 08 K 5/21, C 08 F 299/04

(21) Anmeldenummer: **84114605.3**

(22) Anmeldetag: **01.12.84**

(54) **Neue Amine, Verfahren zu deren Herstellung sowie deren Verwendung in radikalisch polymerisierbaren Massen.**

(30) Priorität: **13.12.83 DE 3345103**

(43) Veröffentlichungstag der Anmeldung: **17.07.85 Patentblatt 85/29**

(45) Bekanntmachung des Hinweises auf die Patenterteilung: **01.04.87 Patentblatt 87/14**

(84) Benannte Vertragsstaaten: **DE FR GB IT SE**

(56) Entgegenhaltungen:
**EP - A - 0 042 991**
**AT - B - 331 518**
**DE - A - 2 336 264**
**US - A - 4 243 763**

(73) Patentinhaber: **BAYER AG, Konzernverwaltung RP Patentabteilung, D-5090 Leverkusen 1 Bayerwerk (DE)**

(72) Erfinder: **Winkel, Jens, Dr., Hahnenweg 6, D-5000 Koeln 80 (DE)**
Erfinder: **Klein, Gerhard, Dr., von-Flotow-Strasse 7, D-4019 Monheim (DE)**
Erfinder: **Meier, Helmut-Martin, Dr., Wodantal 28, D-4320 Hattingen (DE)**
Erfinder: **Süling, Carlhans, Dr., Carl-Leverkus-Strasse 10, D-5068 Odenthal (DE)**
Erfinder: **Arlt, Dieter, Prof. Dr., Rybniker Strasse 2, D-5000 Koeln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 148 401 B1

ACTORUM AG

**Beschreibung**

Die Erfindung betrifft neue, ungesättigte Gruppen aufweisende tertiäre aromatische Amine, Verfahren zu deren Herstellung sowie diese Amine als Aktivatoren enthaltende radikalisch polymerisierbare Massen, insbesondere Dentalmassen.

Die Polymerisation von ethylenisch ungesättigten Verbindungen wie Styrol, Acrylsäure oder Methacrylsäure bzw. mono- oder polyfunktionellen Acrylsäureestern oder Methacrylsäureestern wird durch freie Radikale initiiert. Diese freien Radikale können u.a. von organischen Peroxiden gebildet werden. Bei Verwendung von Diacylperoxiden als Radikalinitiatoren kann der Zerfall der Peroxide durch tertiäre aromatische Amine erheblich beschleunigt werden.

Systeme dieser Art dienen z.B. zur Herstellung von Polymeren auf Basis polyfunktioneller Acrylsäure- oder Methacrylsäureester, die u.a. auf dem Dentalgebiet als Zahnfüllungen oder Zahnersatz oder als Knochenzemente Verwendung finden. In den so hergestellten Polymeren sind jedoch nach der Polymerisationsreaktion noch freies, an die hochmolekularen Ketten chemisch nicht gebundenes Amin und auch Reaktionsprodukte des Amins mit dem Diacylperoxid enthalten.

Die Möglichkeit der Diffusion dieser Substanzen in das Umgebungsmedium kann ein ernsthaftes Hindernis für den Einsatz der Polymeren im Bereich der Medizin darstellen.

Es hat daher nicht an Versuchen gefehlt, Amine zu synthetisieren, die mit den ethylenisch ungesättigten Verbindungen copolymerisierbare Gruppen aufweisen, um so die Amine bzw. deren Reaktionsprodukte chemisch an das Polymerisat zu binden. Ein an sich naheliegender Weg besteht darin, (Meth)acrylsäureester von tertiären Aminoalkoholen herzustellen. Die bis jetzt bekanntgewordenen einpolymerisierbaren (Meth)acrylsäureester von OH-Gruppenhaltigen tertiären Amine besitzen jedoch alle eine wesentlich geringere Aktivität hinsichtlich der Beschleunigung des Peroxidzerfalls als die freien Amine [Fr. Hrbák, V. Hynková; Makromol. Chem. 176, 1669-1678 (1975)].

Überraschenderweise wurde nun gefunden, dass die nachstehend beschriebenen Urethan- oder Harnstoffgruppen aufweisenden tertiären Amine eine mindestens gleich hohe, in der Regel sogar höhere Aktivität als die entsprechenden Aminoalkohole zeigen.

Gegenstand der Erfindung sind tertiäre aromatische Amine der allgemeinen Formel (I):

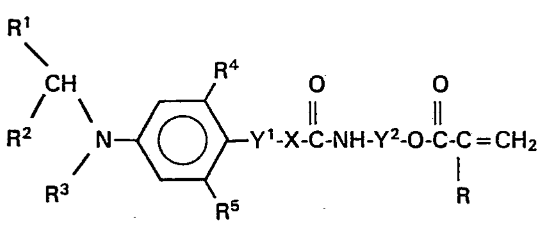

(I)

in welcher

R Wasserstoff oder eine Methylgruppe darstellt,

$Y^1$ für einen gegebenenfalls verzweigten Alkylenrest mit 1 bis 6 C-Atomen, vorzugsweise 1 bis 3 C-Atomen und

$Y^2$ für einen gegebenenfalls verzweigten Alkylenrest mit 2 bis 8 C-Atomen, vorzugsweise 2 bis 5 C-Atomen, stehen,

X Sauerstoff oder eine Gruppe -NH- bedeutet,

$R^1$ und $R^2$ gleich oder verschieden sind und Wasserstoff, eine gegebenenfalls durch eine oder mehrere (vorzugsweise nur eine) Hydroxy-, Amino-, Epoxy-, Urethan-, Harnstoff-, Ester- oder Ethergruppen substituierte Alkyl-, Alkenyl-, Cycloalkyl-, Cycloalkenyl-, Aryl-, Aralkyl- oder Alkarylgruppe mit 1 bis 11 C-Atomen bedeuten oder

$R^1$ und $R^2$ zusammen einen 3- bis 6gliedrigen Ring bilden, welcher gegebenenfalls Stickstoff, Sauerstoff oder Schwefel als Heteroatome enthält,

$R^3$ die Bedeutung von $R^1$ hat oder für eine Gruppe

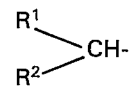

steht oder

$R^2$ und $R^3$ zusammen mit der Gruppe -CH-N- einen 5- oder 6gliedrigen Ring bilden, welcher gegebenenfalls Sauerstoff als weiteres Heteroatom enthält, und

$R^4$ und $R^5$ gleich oder verschieden sind und Wasserstoff, eine gegebenenfalls durch Halogen substituierte Alkyl- oder Alkenylgruppe mit 1 bis 10 C-Atomen oder Halogen darstellen.

Erfindungsgemäss bevorzugt sind Verbindungen, in denen $R^1$ für Wasserstoff steht. Bevorzugt stellt ferner $R^2$ Wasserstoff oder Methyl (insbesondere Wasserstoff) dar; $R^3$ ist vorzugsweise Methyl oder Ethyl, insbesondere Methyl. $R^4$ und $R^5$ stehen vorzugsweise für Wasserstoff oder Methyl.

Gegenstand der Erfindung ist auch ein Verfahren zur Herstellung von Verbindungen der allgemeinen Formel (I), welches dadurch gekennzeichnet ist, dass man tertiäre Amine der allgemeinen Formel (II)

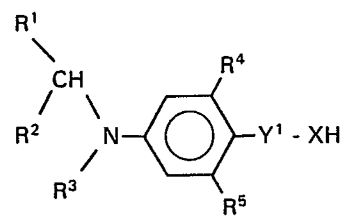

(II)

mit Isocyanaten der allgemeinen Formel (III)

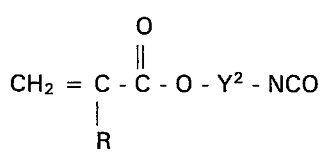

(III)

wobei R, $R^1$ bis $R^5$, $Y^1$, $Y^2$ und X die oben angegebene Bedeutung haben, bei Temperaturen in der Regel zwischen −30 °C und 150 °C, bevorzugt zwischen 0 und 50 °C, gegebenenfalls in einem inerten organischen Lösungsmittel, miteinander umsetzt.

Gegenstand der Erfindung ist weiterhin die Verwendung von Verbindungen gemäss Formel (I) als Aktivatoren in radikalisch polymerisierbaren Massen.

Gegenstand der Erfindung sind auch polymerisierbare Massen, enthaltend ein olefinisch ungesättigtes Monomer, ein tertiäres aromatisches Amin sowie gegebenenfalls organische und/oder anorganische Füllstoffe und/oder andere an sich bekannte Hilfs- und Zusatzstoffe, wobei das Amin eine Verbindung gemäss Formel (I) ist.

Die zur Herstellung der erfindungsgemässen Verbindungen dienenden Amine der Formel (II) bzw. Isocyanate der Formel (III) sind bekannt oder lassen sich nach an sich bekannten Verfahren herstellen.

Die Amine der Formel (II) können nach literaturbekannten Methoden erhalten werden, wie sie z.B. in G. Ferri, Reaktionen der organischen Synthese, Georg Thieme Verlag, Stuttgart (1978) im Kap. 1.3 oder im Kap. 8 beschrieben sind.

Isocyanate der Formel (III) können erhalten werden, indem man gegebenenfalls als Säureaddukte vorliegende Dihydrooxazine mit Phosgen bei −20 bis +20°C in einem mit Wasser nicht mischbaren Lösungsmittel in Gegenwart einer wässrigen Lösung einer Base umsetzt, wobei die Dihydrooxazine die allgemeine Formel

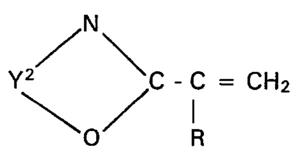

(IV)

aufweisen.

Die Herstellung der als Ausgangsmaterialien einzusetzenden Dihydrooxazine (IV) erfolgt in Analogie zu Verfahren des Standes der Technik. So können die Ausgangsverbindungen (IV) beispielsweise aus N-Hydroxy-methylamiden der allgemeinen Formel

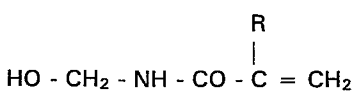

(V)

und einem Olefin nach dem in Liebig's Annalen 697, Seiten 171-180 (1966) beschriebenen Verfahren hergestellt werden.

Vorteilhafter gewinnt man sie aus Formaldehyd, einem Nitril der allgemeinen Formel

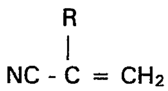

(VI)

und einem Olefin analog dem in Synthesis (1971), Seiten 92-95 beschriebenen Verfahren.

Bei diesem Verfahren wird Formaldehyd in einem Lösungsmittel in Gegenwart äquimolarer Mengen einer starken Säure in einem Temperaturbereich zwischen 30 und 100°C, vorzugsweise bei 50-60°C mit dem Nitril der allgemeinen Formel (VI) umgesetzt.

Das dabei gebildete Amidomethyliumion der Formel

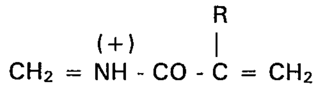

(VII)

reagiert mit dem Olefin in einer polaren Cycloaddition zu dem Säureaddukt des Dihydrooxazins, aus welchem das als Ausgangsmaterial geeignete Dihydrooxazin (IV) durch an sich bekannte Behandlung mit einer Base gewonnen werden kann.

Formaldehyd kann dabei entweder durch Depolymerisation aus Paraformaldehyd oder aus 1,3,5-Trioxan gewonnen werden. Als Lösungsmittel können Carbonsäuren, Carbonsäureanhydride, Ether wie z.B. Tetrahydrofuran, Dioxan, Glyme, Diglyme, Amide wie z.B. N-Methylpyrrolidon, Harnstoff, wie z.B. 1,3-Dimethylpyrrolidon-2 oder Sulfolan verwendet werden. Bevorzugt sind Carbonsäuren, insbesondere Essigsäure.

Als starke Säuren kommen Schwefelsäure, Phosphorsäure, Chlorwasserstoff, Fluorwasserstoff, Borfluorwasserstoffsäure und Sulfonsäuren in Betracht. Am vorteilhaftesten wird Schwefelsäure benutzt. In jedem Falle ist auf Wasserausschluss zu achten.

Das Nitril wird in äquimolaren Mengen in einem Temperaturbereich zwischen 30 und 100°C, vorzugsweise bei 50-60°C, zu einer Lösung von Formaldehyd und der starken Säure in dem Lösungsmittel gegeben. Als Olefin bzw. Olefingemisch kommen beispielsweise cis-Buten-2, trans-Buten-2 und 1-Buten, gegebenenfalls im Gemisch, in Betracht. Derartige technische Buten-Gemische, die neben reaktionsfähigen Butenen noch inerte Butane enthalten, fallen beispielsweise bei der destillativen Auftrennung der Spaltprodukte von Benzincrackern als $C_4$-Fraktion an. Andere grosstechnische $C_4$-Fraktionen von Benzincrackern mit einem hohen Gehalt an iso-Buten gestatten die Herstellung von Dihydrooxazin-Gemischen mit einem hohen Gehalt an 6,6-Dimethyl-substituierten Isomeren. Da das in den genannten Fraktionen enthaltene 1-Buten im Vergleich zu cis- und trans-Buten-2 reaktionsträger ist, ist der Gehalt an 6-Ethyl-2-vinyl-5,6-dihydrooxazin in den Dihydrooxazin-Gemischen im allgemeinen geringer als der Gehalt der eingesetzten $C_4$-Fraktion an 1-Buten.

Die Reaktion zwischen dem Säureaddukt des Dihydrooxazins und dem Olefin bzw. Olefingemisch kann in einem offenen Gefäss unter Durchleiten oder Zutropfen des Olefins, bei gasförmigen Olefinen auch unter Druck durchgeführt werden.

Die Bildung des Dihydrooxazins aus dem Amidomethyliumion (VII) und dem Olefin erfolgt in einer stereospezifischen cis-Addition [siehe Chem. Ber. 103, 3242 (1970)]. Aus einem cis-/trans-Olefin-Gemisch bildet sich deshalb ein entsprechendes cis-/trans-Gemisch des 5,6-Dimethyldihydrooxazins.

Wie bereits erwähnt, kann das freie Dihydrooxazin aus dem erhaltenen Säureaddukt in an sich bekannter Weise mittels einer Base wie z.B. Natriumhydroxid oder Kaliumhydroxid freigesetzt werden. Es ist jedoch auch möglich, die Dihydrooxazine in Form ihrer Säureaddukte einzusetzen.

Die Phosgenierung der Dihydrooxazine bzw. Dihydrooxazin-Gemische erfolgt vorzugsweise nach der bekannten Zweiphasen-Phosgenierung, wie sie beispielsweise in DE-AS 1 924 535 für die Phosgenierung von Oxazolinen oder von Dihydrooxazinen beschrieben ist. Dabei werden pro Mol Dihydrooxazin bzw. pro Mol Säureaddukt des Dihydrooxazins bzw. Dihydrooxazin-Gemischs im allgemeinen 1 bis 2 Mol Phosgen und pro Mol Phosgen mindestens 2 Mol einer wässrigen Base eingesetzt. Falls Säureaddukte der Dihydrooxazine eingesetzt werden, benötigt man zusätzlich noch eine der Säure äquivalente Menge an Base.

Als Basen können wässrige Lösungen von Alkalihydroxiden und -carbonaten verwendet werden. Bevorzugt ist wässrige Natronlauge. Das Dihydrooxazin und das Phosgen werden im allgemeinen als Lösungen in einem unpolaren, mit Wasser nicht mischbaren Lösungsmittel eingesetzt. Dafür kommen Kohlenwasserstoffe, Halogenkohlenwasserstoffe wie z.B. Methylenchlorid, Chloroform, 1,2-Dichlorpropan, Chlorbenzol und Dichlorbenzol, Ester wie z.B. Ethylacetat oder Ether wie Diethyl- oder Dibutylether in Frage. Am vorteilhaftesten ist die Verwendung von Halogenkohlenwasserstoffen, insbesondere von Methylenchlorid.

Die Lösungen des Dihydrooxazins, des Phosgens und der Base werden gleichzeitig und gleichmässig dem Reaktionsgefäss zugeführt. Dabei ist für eine intensive Durchmischung zu sorgen. Die Temperatur wird bei −20 bis +20°C, vorzugsweise zwischen 0 und 5°C gehalten. Da die Reaktion sehr schnell abläuft, ist eine kontinuierliche Reaktionsführung zweckmässig.

Weitere Synthesemethoden für die Isocyanate der Formel (III) sind z.B. in US-PS 2 718 516 und US-PS 2 821 544 beschrieben.

Die in den erfindungsgemässen Massen zu verwendenden Monomeren weisen mindestens eine radikalisch polymerisierbare Doppelbindung auf. Vorzugsweise werden Monomere mit mehr als einer Doppelbindung und Siedepunkten über 100°C bei 13 mbar, allein oder gegebenenfalls im Gemisch mit monofunktionellen Monomeren verwendet. Dadurch werden hochvernetzte Polymerisate oder Copolymerisate erhalten. Die Molgewichte der Monomeren können zwischen etwa 70 und 20000 liegen, vorzugsweise zwischen etwa 150 und 1000. Die Viskosität der Monomeren kann durch geeignete Abmischung von höherviskosen bzw. höhermolekularen Monomeren mit niedrigviskosen Monomeren eingestellt werden. Die Monomeren enthalten gegebenenfalls geringe Mengen an Polymerisationsinhibitoren, wie z.B. 0,01-0,2% 2,6-Di-t-butyl-p-kresol.

Als erfindungsgemäss polymerisierbare Monomere kommen beispielsweise in Frage:

Ester von ungesättigten Mono- oder Dicarbonsäuren, z.B. Ester der Acryl-, Methacryl-, α-Cyanacryl-, Croton-, Zimt-, Sorbin-, Malein-, Fumar- oder Itaconsäure mit aliphatischen, cycloaliphatischen oder aromatisch-aliphatischen ein- bis vierwertigen Alkoholen mit 2-30 Kohlenstoffatomen, z.B. Methyl (meth-)acrylat, n-, i- und t-Butyl (meth-)acrylat, 2-Ethylhexylacrylat, Laurylacrylat, Dihydrodicyclopentadienyl-(meth-)acrylat, Dihydroxymethyl-tricyclo[5,2,1,0,$^{2,6}$] decandi(meth)-acrylat gemäss der DE-PS 2 200 021, Methylglycoldi-(meth)acrylat, Hydroxyethyl(meth)acrylat, Hydroxypropyl(meth)-acrylat, Ethylenglycoldiacrylat, Diethylenglycoldiacrylat, Triethylenglycoldi(meth)acrylat, Neopentylglycoldi(meth)acrylat, 1,4-Dimethylolcyclohexandiacrylat, Pentaerythrit-tri- und tetra(meth)acrylat, Trimethylolpropan tri(meth)acrylat, Ethyl-α-cyanoacrylat, Ethylcrotonat, Ethylsorbinat, Diethylmaleinat, Diethylfumarat sowie das Di(meth)acrylat des oxalkylierten Bisphenol A gemäss US-PSS 3 810 938 und 3 923 740, Di(meth)acrylsäureester von oxalkyliertem Trimethylolpropan oder Pentaerythrit gemäss US-PS 3 380 831 und auch die (Meth)acrylester von oxyalkylierten Di-(hydroxymethyl)-tricyclo[5,2,1,0$^{2,6}$]-decanen, wie sie in DE-OS 2 931 925 und DE-OS 2 931 926 beschrieben werden.

Weitere in den erfindungsgemässen Massen einsetzbare Monomere sind Amide der (Meth)acrylsäure, die gegebenenfalls am Stickstoffatom mit Alkyl-, Alkoxyalkyl- oder Hydroxyalkylresten substituiert sein können, wie z.B. N-Isobutylacrylamid, Diacetonacrylamid, N-Methylolacrylamid, N-Methoxymethylacrylamid, N-Butoxymethylmethacrylamid, Ethylenglycolbis-(N-methylolacrylamid)-ether und Methylen-bis-acrylamid; Triacrylformal; Vinylester von Mono- und Dicarbonsäuren mit 2 bis 20 Kohlenstoffatomen, z.B. Vinylacetat, Vinylpropionat, 2-Ethylhexansäure-vinylester, Versaticsäurevinylester und Divinyladipat; Vinylether von ein- oder zweiwertigen Alkoholen mit 3 bis 20 Kohlenstoffatomen, z.B. Isobutylvinylether, Octadecylvinylether, Ethylenglycoldivinylether und Diethylenglycoldivinylether; Mono-N-Vinylverbindungen, z.B. N-Vinylpyrrolidon, N-Vinylpiperidon, N-Vinylcaprolactam, N-Vinylmorpholin, N-Vinyloxazolidon, N-Vinylsuccinimid, N-Methyl-N-vinylformamid und N-Vinylcarbazol; Allylether und -ester, z.B. Trimethylolpropandiallylether, Trimethylolprop-triallylether, Allyl(meth-)acrylat, Diallylmaleinat, Diallylphthalat und dessen Präpolymere sowie beliebige Gemische aller aufgeführten ungesättigten Verbindungen.

Für medizinische Zwecke sind die Epoxidacrylate und Urethanacrylate besonders geeignet. Als Beispiele für solche Verbindungen seien aufgeführt:

a) Reaktionsprodukte aus monofunktionellen Epoxiden und (Meth)acrylsäuren gemäss US-PS 2 484 487 und US-PS 2 575 440;

b) Reaktionsprodukte aus bifunktionellen Epoxiden und ungesättigten Fettsäuren nach US-PS 2 456 408;

c) Reaktionsprodukte aus polyfunktionellen aromatischen oder aliphatischen Glycidethern und (Meth-)acrylsäure gemäss US-PSS 3 179 623, 3 066 112, 2 824 851, und DE-PS 1 644 817;

d) Reaktionsprodukte aus Epoxidharzen und (Meth)acrylsäurechlorid gemäss US-PS 3427161 und US-PS 2 890 202;

e) ungesättigte Polyurethane (Urethanacrylate) und Polyharnstoffe aus Hydroxyalkyl(meth)acrylaten, Aminoalkyl(meth)acrylaten und gegebenenfalls Polyolen oder Polyaminen, wie sie in den US-PSS 3 425 988, 3 709 866, 3 629 187, 4 089 763 und

4110184 sowie DE-PSS 1644798, 1644797, DOS 2357402, 2357324, 2358948 beschrieben sind.

Weitere Beispiele geeigneter Comonomere sind der nachstehenden Zusammenstellung zu entnehmen; in den Strukturformeln stehen

R für $CH_2 = C - CO -$ oder $CH_2 = CH - CO-$
|
$CH_3$

R' für H oder $CH_2 - OR$
n für eine Zahl zwischen 1 und 4 und
m für eine Zahl zwischen 0 und 4

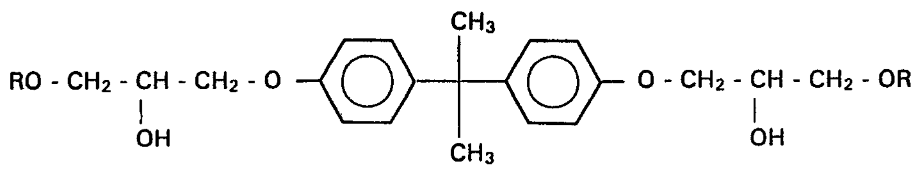

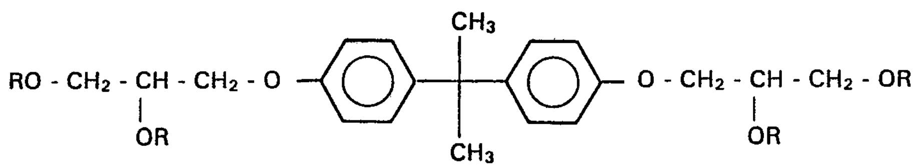

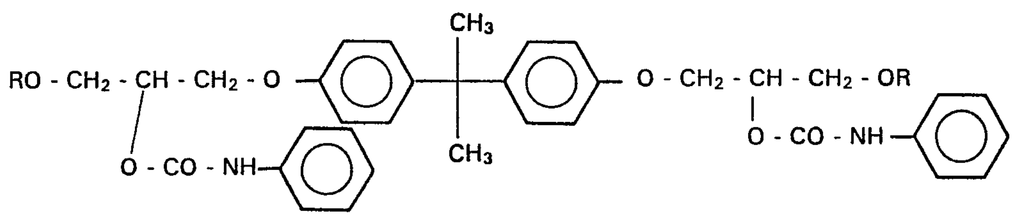

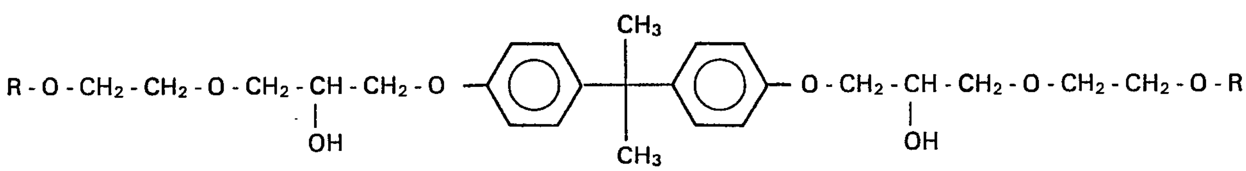

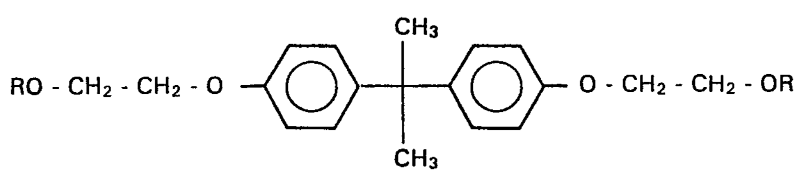

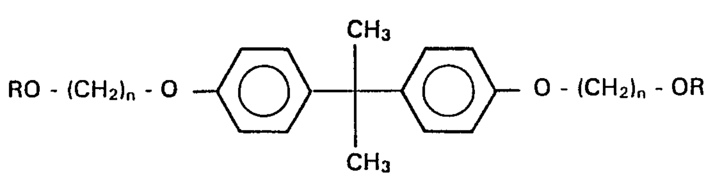

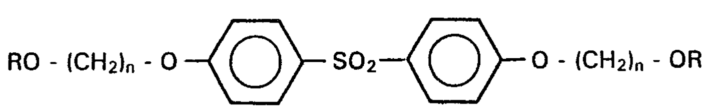

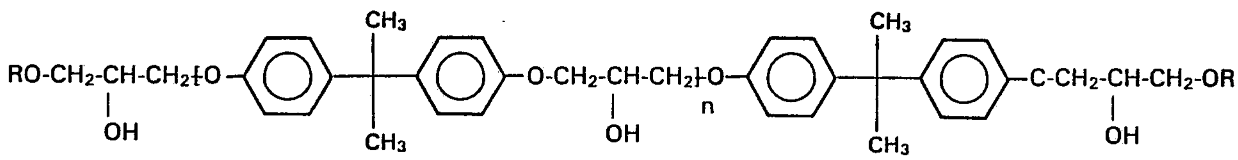

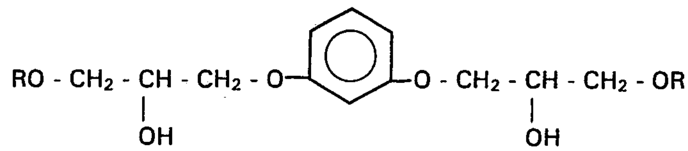

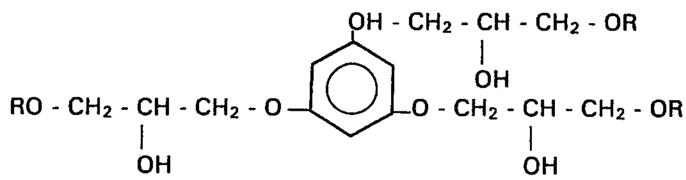

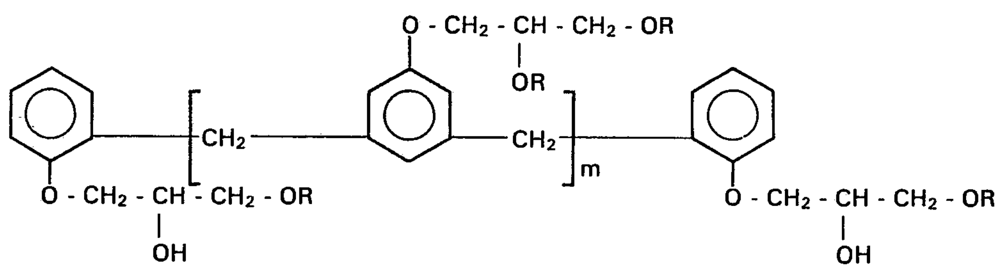

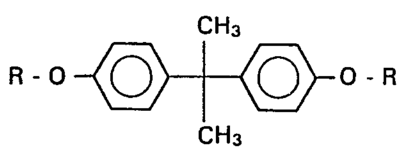

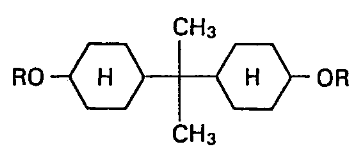

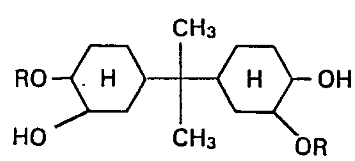

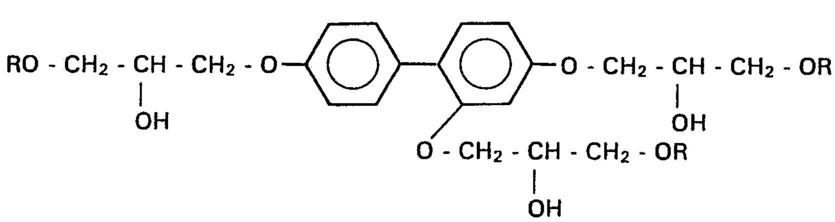

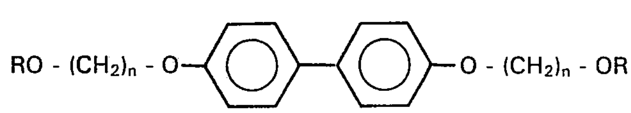

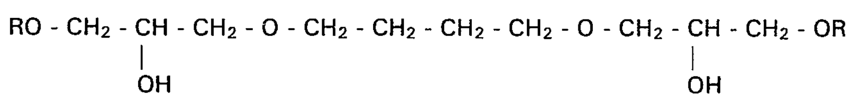

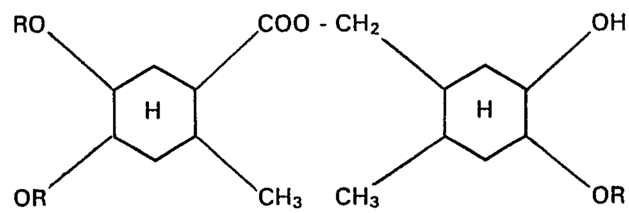

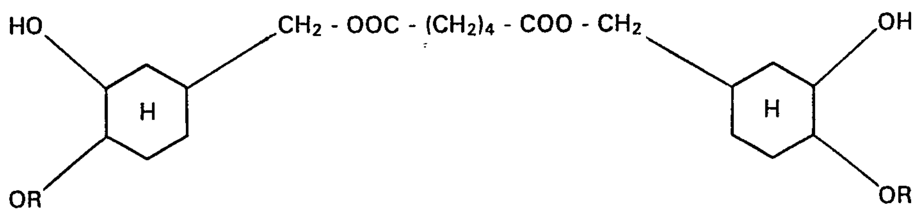

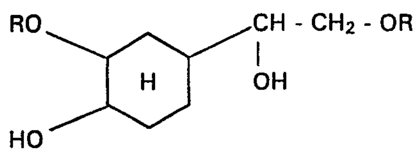

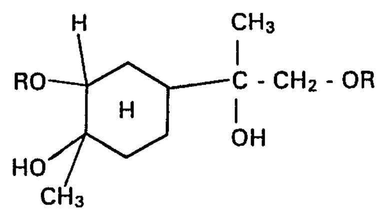

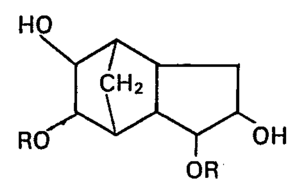

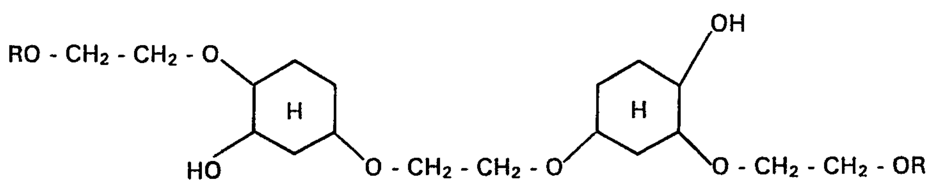

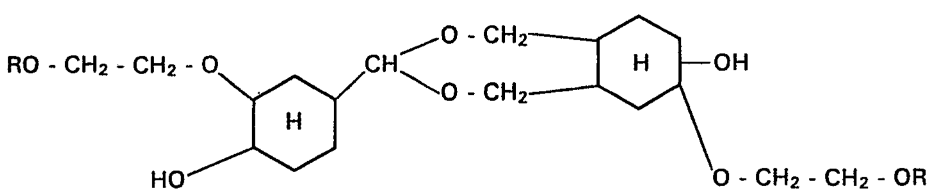

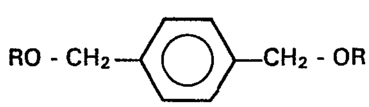

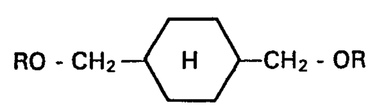

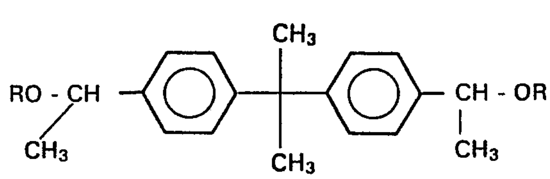

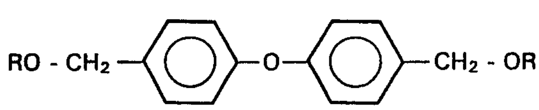

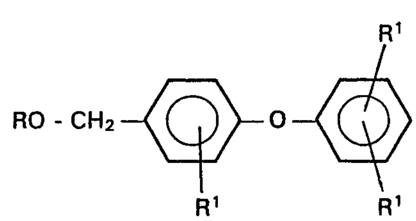

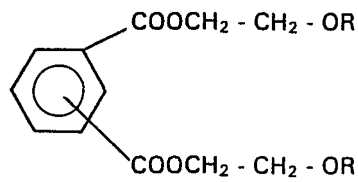

in der ortho-, meta- oder para-Form

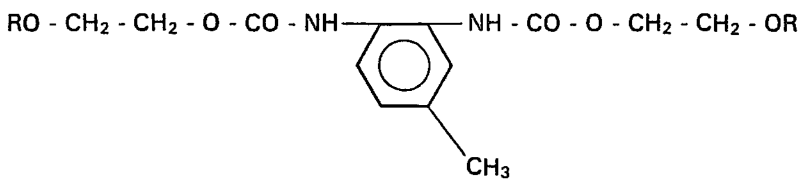

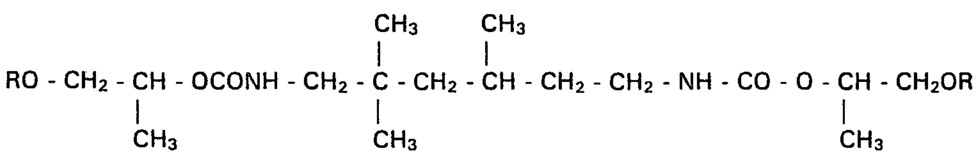

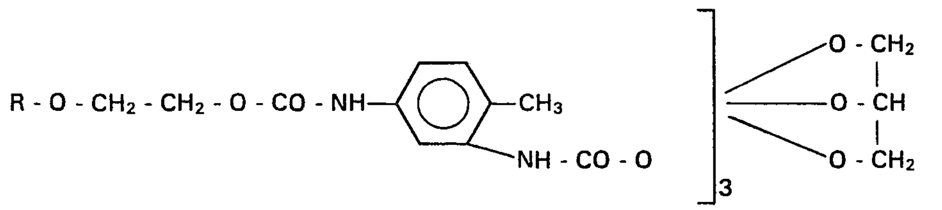

sowie Verbindungen der allgemeinen Formel

$$R - (O - D - O - X)_n - OD - OR$$

wobei HO-D-OH ein Polyol und HO-X-OH eine Dicarbonsäure darstellen, die jeweils gesättigt oder ungesättigt, cyclisch oder acyclisch sein können.

Je nach Anwendungszweck können in den erfindungsgemässen Massen noch andere Stoffe mitverwendet werden, wie z.B. anorganische und/oder organische Füllstoffe und Pigmente, Stabilisatoren, Farbstoffe, spezielle Lichtschutzmittel, Fluoreszenzmittel, Weichmacher sowie lösliche, quellbare oder unlösliche hochmolekulare Verbindungen.

Die Teilchengrösse von Füllstoffen kann z.B. zwischen 10 nm und ca. 50 µm liegen. Als anorganische Füllstoffe kommen z.B. Metall-Oxyde, Silikate, Phosphate, Sulfate, Carbonate und Fluoride in Frage. Im einzelnen seien aufgeführt: Bergkristall, Quarzit, Novakulit, Cristobalit, Quarzglas, hochdisperse Kieselsäure, Aluminiumoxid, Zirkondioxid, Titandioxid, Bariumsulfat, Calciumfluorid, Barium- oder Calciumsilikate, β-Eukryptit, Spodumen, Borsilikatgläser, Glaskeramiken, z.B. auf der Basis von µ-Cordierit, sowie Lanthan und Zirkon enthaltende Glaskeramiken gemäss DE-OS 2 347 591.

Die anorganischen Füllstoffe werden vorzugsweise mit einem Haftvermittler vorbehandelt, um den Verbund zur Polymermatrix zu erhöhen. Hierzu sind besonders Silane wie Trimethoxy-(3-methacrylyloxypropyl)-silan oder Titansäureester geeignet.

Als organische feinteilige Füllstoffe werden z.B. Polymere verwendet, welche durch Polymerisation von Vinylmonomeren, Pfropfpolymerisation, Polyaddition oder Polykondensation hergestellt worden sind. Im allgemeinen werden Vinylpolymerisate eingesetzt, welche durch Masse-, Suspensions-, Emulsions- oder Fällungspolymerisation hergestellt wurden. Der Quellungsgrad der Polymerisate kann durch Einpolymerisieren von polyfunktionellen Monomeren herabgesetzt werden.

Als geeignete Polymerisate seien beispielhaft Homo- bzw. Copolymerisate aus (Meth)acrylsäureestern, wie Methylmethacrylat, Ethylacrylat, Butylmethacrylat, Dodecylmethacrylat, Ethylenglycoldimethacrylat, Triethylenglycol-dimethacrylat, Bisphenoldiglycidyldimethacrylat und 1,12-Dodecandiol-dimethacrylat genannt. Gut geeignet sind auch Polymerisate, die gemäss DE-OS 28 49 280 erhalten wurden.

Die organischen feinteiligen Füllstoffe können auch anorganische mikrofeine Substanzen in feinverteilter Form enthalten. Hybridfüllstoffe lassen sich als Splitterpolymerisate erhalten, indem Mischungen aus Monomer und mikrofeinen anorganischen Substanzen in Masse polymerisiert und anschliessend durch Mahlung zerkleinert werden. In

Perlform können derartige Hybridfüllstoffe gemäss DE-OS 2849936 hergestellt werden.

Als Radikalbildner kommen für die erfindungsgemässen Massen alle an sich bekannten organischen Peroxide in Betracht.

Bevorzugte Initiatoren sind z.B. Diacetylperoxid, Dibenzoylperoxid, Bis(4-chlorbenzoyl)peroxid, Bis-(2-methylbenzoyl)peroxid, Phthaloylperoxid, Succinylperoxid, Dilauroylperoxid, Acetylcyclohexansulfonylperoxid, Isopropylpercarbonat, Cyclohexylpercarbonat und Bis(4-tert.-butylcyclohexyl)percarbonat. Andere geeignete Initiatoren sind Peroxyester wie tert.-Butylperoxyacetat, tert.-Butylperoxybenzoat, tert.-Butylperoctoat, Dicyclohexylperoxydicarbonat oder 2,5-Dimethylhexan-2,5-diperoctoat, Alkylperoxide wie Bis-(tert.-butylperoxybutan), Dicumylperoxid, tert.-Butylcumylperoxid, Hydroperoxide wie Cumolhydroperoxid, tert.-Butylhydroperoxid, Cyclohexanonhydroperoxid, Methylethylketonhydroperoxid, Perketale oder Ketonperoxide wie Acetylacetonperoxid.

Die erfindungsgemässen Verbindungen werden bevorzugt in einer Konzentration von 0,1-5 Gew.-%, bezogen auf Gesamtmenge an olefinisch ungesättigten Monomeren, dem zu polymerisierenden Reaktionsgemisch zugefügt. Besonders bevorzugt werden 0,2-2 Gew.-% verwendet.

Das als Radikalbildner dienende organische Peroxid sollte wie üblich in einer Konzentration von 0,2 bis 4 Gew.-%, vorzugsweise 0,5 bis 2 Gew.-%, bezogen auf Monomer, anwesend sein.

Die erfindungsgemässen Verbindungen können im Prinzip in Mischung mit jeder anderen Komponente (ausgenommen dem Peroxid) der polymerisierbaren Masse eingesetzt werden. Vorzugsweise geht man jedoch (insbesondere bei Anwesenheit von Füllstoffen in der polymerisierbaren Masse) gemäss der Lehre von US-PS 3926906 so vor, dass man das tertiäre Amin mit ca. der Hälfte des polymerisierbaren Monomeren und etwa der Hälfte des gegebenenfalls mitverwendeten Füllstoffs zu einer «Aktivatorpaste» vermischt, während aus dem Peroxid sowie dem Rest von Monomer und Füllstoff eine «Katalysatorpaste» hergestellt wird. Unmittelbar vor der Anwendung mischt man dann Aktivator- und Katalysatorpaste im Verhältnis von etwa 1:1, worauf innerhalb kurzer Zeit die Masse durch Polymerisation aushärtet.

In der Regel enthält die Aktivatorpaste neben dem oder den Monomeren bis zu 90 Gew.-%, vorzugsweise 50 bis 85 Gew.-%, bezogen auf gesamte Paste, eines der obengenannten organischen und/oder anorganischen Füllstoffe, 0,1 bis 20 Gew.-%, bezogen auf Monomer(e), des tertiären Amins und gegebenenfalls bis zu 5 Gew.-%, bezogen auf gesamte Paste, an Hilfs- und Zusatzstoffen (z.B. Lichtschutzmittel, Antioxydantien oder Farbstoffe).

Da die polymerisierbaren Aktivatoren der Formel (I) in den Polymerverbund eingebaut werden, sind sie besonders für die Herstellung von mit dem menschlichen Körper in Kontakt kommenden Polymerisaten geeignet, z.B. von Knochenzementen, Dentalzementen, Zahnfüllmassen und medizinischen Versiegelungsmassen. Darüberhinaus sind sie auch infolge ihrer geringen Basizität nicht gewebereizend.

*Beispiel 1*

A) 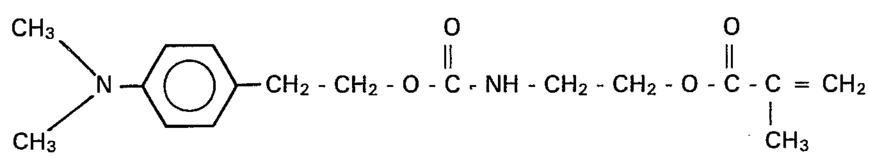

1,5 g 4-Hydroxyethyl-N,N-dimethylanilin werden mit 1,4 g Isocyanatoethylmethacrylat in Chloroform umgesetzt. Nach Abdestillieren des Lösungsmittels erhält man 3,8 g eines weissen Feststoffes, der durch Chromatographie an Kieselgel mit Toluol/Essigester (7:3) weiter gereinigt werden kann.

Schmelzpunkt: 72 bis 74°C.

$^1$H-NMR: δ = 1.92(3H), 2.62-2.92(2H); 2.92(6H); 3.35-3.68(2H); 3.98-4.38(4H); 4.90(1H); 5.52-6.15(2H); 6.62-7.18(4H)

In analoger Weise werden aus den entsprechenden Alkoholen bzw. Aminen und Isocyanato(meth)acrylsäureestern die folgenden Verbindungen erhalten:

B) 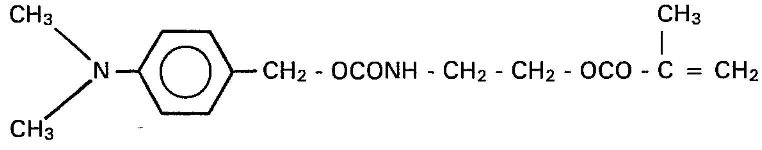

$^1$H-NMR: δ = 1.90(3H); 2.90(6H); 3.40-4.40(4H); 5.05(2H); 5.20(1H); 5.50-6.10(2H); 6.65-7.15(4H)

C) 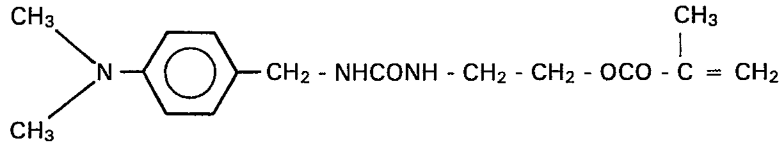

$^1$H-NMR: δ = 1.92(3H), 2.85(6H); 3.10-4.20(6H); 5.40-5.70(2H); 5.40-6.10(2H); 6.5-7.15(4H)

D) 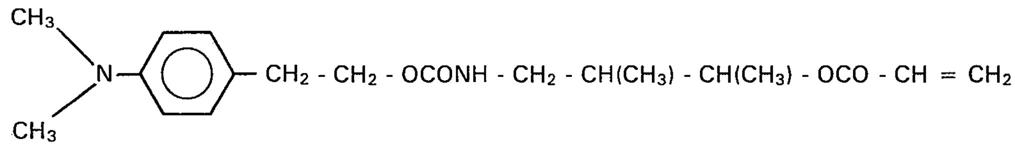

$^1$H-NMR: δ = 0.80-1.35(6H); 1.55-2.10(1H); 2.70-3.30(4H); 2.85(6H); 4.10-4.40(2H); 4.70-5.20(2H); 5.60-6.30(3H); 6.60-7.25(4H)

E) 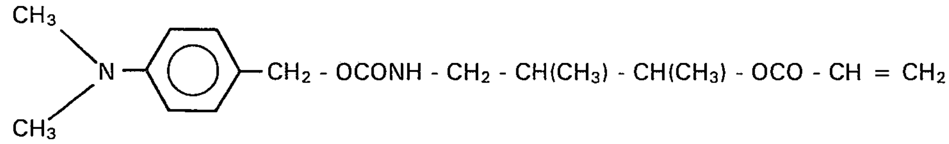

$^1$H-NMR: δ = 0.80-1.35(6H); 1.50-2.05(1H); 2.85(6H); 3.05-3.25(2H); 4.70-5.25(2H); 4.90(2H); 5.60-6.30(3H); 6.60-7.30(4H)

F) 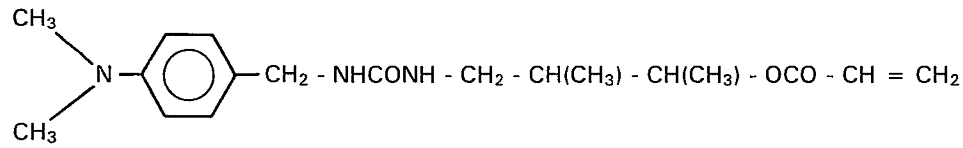

$^1$H-NMR: δ = 0.75-1.25(6H); 1.45-2.00(1H); 2.5-3.15(2H); 2.85(6H); 4.00-4.20(2H); 4.65-5.10(1H); 5.60-6.60(5H); 6.55-7.05(4H)

*Beispiel 2*

In 100 g einer Mischung aus 70 Gew.-Teilen Bis-GMA und 30 Gew.-Teilen Triethylenglykoldimethacrylat werden jeweils 0,0025 Mol Benzoylperoxid bzw. 0,0025 Mol Amin gelöst. Je 2 ml Peroxidlösung und Aminlösung werden miteinander gemischt.

Die Temperatur während der Härtung wird gemessen und die Härtungszeit (Zeit bis zum Erreichen des Temperaturmaximums) bestimmt.

| Amin | Härtungszeit | |
|---|---|---|
| 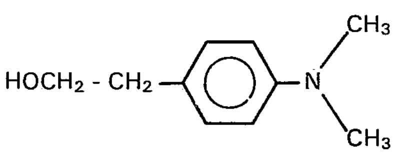 (Vergleichsversuch) | 3 min | 30 sec |
| A | 2 min | 30 sec |
| D | 2 min | |

*Beispiel 3*

In einer Mischung aus 62 Gew.-Teilen Bis-GMA und 38 Gew.-Teilen Triethylenglykoldimethacrylat werden 0,04% Jonol und 2,6% ® Tinuvin P gelöst.

In 100 g dieser Lösung werden jeweils 0,007 Mol des Amins D bzw. des Vergleichsversuchs aus Beispiel 2 gelöst. Je 10 g silanisierte Glaskeramik werden mit jeweils 4 g dieser Lösungen zu Pasten verarbeitet.

In einer Mischung aus 62 Gew.-Teilen Bis-GMA und 38 Gew.-Teilen Triethylenglykoldimethacrylat wird 0,04% Jonol gelöst.

In 100 g dieser Lösung wird 0,007 Mol Benzoylperoxid gelöst und wie die Aminlösungen zu einer Paste verarbeitet. Mischt man gleiche Teile der Aminpaste, die das Amin des Vergleichsversuchs enthält, und der Peroxidpaste, so härtet die Mischung in 3 min und 10 sec. Mischt man gleiche Teile der Aminpaste, die das Amin D enthält, mit der Peroxidpaste, so härtet die Mischung in 1 min 30 sec.

**Patentansprüche**

1. Verbindungen der allgemeinen Formel

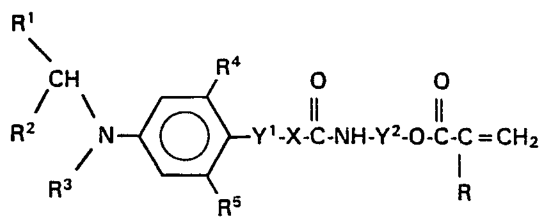 (I)

in welcher

R Wasserstoff oder eine Methylgruppe darstellt,

$Y^1$ für einen gegebenenfalls verzweigten Alkylenrest mit 1 bis 6 C-Atomen, vorzugsweise 1 bis 3 C-Atomen und

$Y^2$ für einen gegebenenfalls verzweigten Alkylenrest mit 2 bis 8 C-Atomen, vorzugsweise 2 bis 5 C-Atomen, stehen,

X Sauerstoff oder eine Gruppe -NH- bedeutet,

$R^1$ und $R^2$ gleich oder verschieden sind und Wasserstoff, eine gegebenenfalls durch eine oder mehrere (vorzugsweise nur eine) Hydroxy-, Amino-, Epoxy-, Urethan-, Harnstoff-, Ester- oder Ethergruppen substituierte Alkyl-, Alkenyl-, Cycloalkyl-, Cycloalkenyl-, Aryl-, Aralkyl- oder Alkarylgruppe mit 1 bis 11 C-Atomen bedeuten oder

$R^1$ und $R^2$ zusammen einen 3- bis 6gliedrigen Ring bilden, welcher gegebenenfalls Stickstoff, Sauerstoff oder Schwefel als Heteroatome enthält,

$R^3$ die Bedeutung von $R^1$ hat oder für eine Gruppe

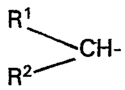

steht oder $R^2$ und $R^3$ zusammen mit der Gruppe -CH-N- einen 5- oder 6gliedrigen Ring bilden, welcher gegebenenfalls Sauerstoff als weiteres Heteroatom enthält, und

$R^4$ und $R^5$ gleich oder verschieden sind und Wasserstoff, eine gegebenenfalls durch Halogen substituierte Alkyl- oder Alkenylgruppe mit 1 bis 10 C-Atomen oder Halogen darstellen.

2. Verbindungen nach Anspruch 1, dadurch gekennzeichnet, dass der Rest $R^1$ für Wasserstoff steht.

3. Verbindungen nach Anspruch 1 oder 2, dadurch gekennzeichnet, dass $R^2$ Wasserstoff oder Methyl darstellt.

4. Verbindungen nach Anspruch 1 bis 3, dadurch gekennzeichnet, dass $R^3$ für Methyl oder Ethyl steht.

5. Verbindungen nach Anspruch 1 bis 4, dadurch gekennzeichnet, dass $R^4$ und $R^5$ für Wasserstoff oder Methyl stehen.

6. Verbindungen nach Anspruch 5, dadurch gekennzeichnet, dass sowohl $R^4$ als auch $R^5$ für Wasserstoff stehen.

7. Verfahren zur Herstellung von Verbindungen nach Ansprüchen 1 bis 6, dadurch gekennzeichnet, dass man tertiäre Amine der allgemeinen Formel

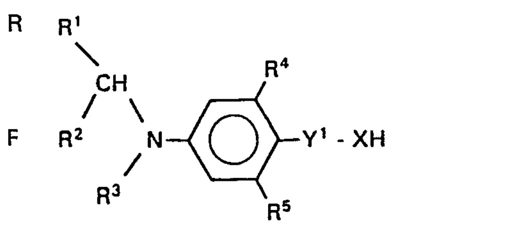

(II)

mit Isocyanaten der allgemeinen Formel

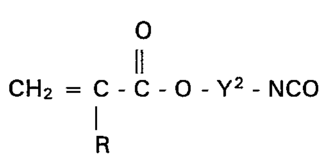

(III)

wobei

R Wasserstoff oder eine Methylgruppe darstellt,

$Y^1$ für einen gegebenenfalls verzweigten Alkylenrest mit 1 bis 6 C-Atomen, vorzugsweise 1 bis 3 C-Atomen und

$Y^2$ für einen gegebenenfalls verzweigten Alkylenrest mit 2 bis 8 C-Atomen, vorzugsweise 2 bis 5 C-Atomen, stehen,

X Sauerstoff oder eine Gruppe -NH- bedeutet,

$R^1$ und $R^2$ gleich oder verschieden sind und Wasserstoff, eine gegebenenfalls durch eine oder mehrere (vorzugsweise nur eine) Hydroxy-, Amino-, Epoxy-, Urethan-, Harnstoff-, Ester- oder Ethergruppen substituierte Alkyl-, Alkenyl-, Cycloalkyl-, Cycloalkenyl-, Aryl-, Aralkyl- oder Alkarylgruppe mit 1 bis 11 C-Atomen bedeuten oder

$R^1$ und $R^2$ zusammen einen 3- bis 6gliedrigen Ring bilden, welcher gegebenenfalls Stickstoff, Sauerstoff oder Schwefel als Heteroatome enthält,

$R^3$ die Bedeutung von $R^1$ hat oder für eine Gruppe

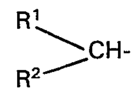

steht oder $R^2$ und $R^3$ zusammen mit der Gruppe -CH-N- einen 5- oder 6gliedrigen Ring bilden, welcher gegebenenfalls Sauerstoff als weiteres Heteroatom enthält, und

$R^4$ und $R^5$ gleich oder verschieden sind und Wasserstoff, eine gegebenenfalls durch Halogen substituierte Alkyl- oder Alkenylgruppe mit 1 bis 10 C-Atomen oder Halogen darstellen,

bei Temperaturen in der Regel zwischen −30°C und 150°C, bevorzugt zwischen 0 und 50°C, gegebenenfalls in einem inerten organischen Lösungsmittel, miteinander umsetzt.

8. Verwendung von Verbindungen gemäss Ansprüchen 1 bis 6 als Aktivatoren bei der radikalischen Polymerisation von olefinische Gruppen aufweisenden Monomeren.

9. Verwendung nach Anspruch 8, dadurch gekennzeichnet, dass 0,1 bis 5 Gew.-% Aktivator, bezogen auf Monomer(e) eingesetzt werden.

10. Polymerisierbare Masse, enthaltend ein olefinisch ungesättigtes Monomer, ein tertiäres Amin als Aktivator sowie gegebenenfalls organische und/oder anorganische Füllstoffe und/oder weitere an sich bekannte Hilfs- und Zusatzstoffe, dadurch gekennzeichnet, dass der Aktivator eine Verbindung gemäss Anspruch 1 bis 6 ist.

**Claims**

1. Compounds of the general formula

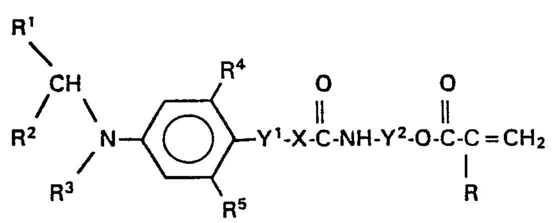

(I)

in which

R represents hydrogen or a methyl group,

$Y^1$ represents an optionally branched alkylene radical with 1 to 6 C atoms, preferably 1 to 3 C atoms,

$Y^2$ represents an optionally branched alkylene radical with 2 to 8 C atoms, preferably 2 to 5 C atoms,

X denotes oxygen or an -NH- group,

$R^1$ and $R^2$ are identical or different and denote hydrogen, or an alkyl, alkenyl, cycloalkyl, cycloalkenyl, aryl, aralkyl or alkylaryl group which has 1

to 11 C atoms and is optionally substituted by one or more (preferably only one) hydroxyl, amino, epoxide, urethane, urea, ester or ether groups, or

$R^1$ and $R^2$ together form a 3-membered to 6-membered ring, which optionally contains nitrogen, oxygen or sulphur as hetero-atoms,

$R^3$ has the meaning of $R^1$ or represents a group

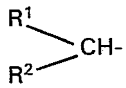

or

$R^2$ and $R^3$, together with the -CH-N- group, form a 5-membered or 6-membered ring, which optionally contains oxygen as a further hetero-atom, and

$R^4$ and $R^5$ are identical or different and represent hydrogen, an alkyl or alkenyl group which has 1 to 10 C atoms and is optionally substituted by halogen, or halogen.

2. Compounds according to Claim 1, characterised in that the radical $R^1$ represents hydrogen.

3. Compounds according to Claim 1 or 2, characterised in that $R^2$ represents hydrogen or methyl.

4. Compounds according to Claims 1 to 3, characterised in that $R^3$ represents methyl or ethyl.

5. Compounds according to Claims 1 to 4, characterised in that $R^4$ and $R^5$ represent hydrogen or methyl.

6. Compounds according to Claim 5, characterised in that both $R^4$ and $R^5$ represent hydrogen.

7. Process for the preparation of compounds according to Claims 1 to 6, characterised in that tertiary amines of the general formula

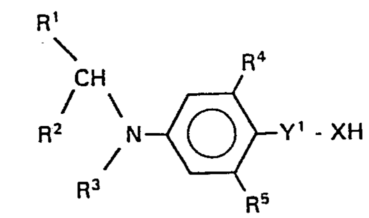

(II)

are reacted with isocyanates of the general formula

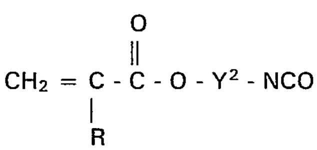

(III)

wherein

R represents hydrogen or a methyl group,

$Y^1$ represents an optionally branched alkylene radical with 1 to 6 C atoms, preferably 1 to 3 C atoms and

$Y^2$ represents an optionally branched alkylene radical with 2 to 8 C atoms, preferably 2 to 5 C atoms,

X denotes oxygen or an -NH- group,

$R^1$ and $R^2$ are identical or different and denote hydrogen, or an alkyl, alkenyl, cycloalkyl, cycloalkenyl, aryl, aralkyl or alkylaryl group which has 1 to 11 C atoms and is optionally substituted by one or more (preferably only one) hydroxyl, amino, epoxide, urethane, urea, ester or ether groups, or

$R^1$ and $R^2$ together form a 3-membered to 6-membered ring, which optionally contains nitrogen, oxygen or sulphur as hetero-atoms,

$R^3$ has the meaning of $R^1$ or represents a group

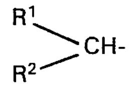

or

$R^2$ and $R^3$, together with the -CH-N- group, form a 5-membered or 6-membered ring, which optionally contains oxygen as a further hetero-atom, and

$R^4$ and $R^5$ are identical or different and represent hydrogen, an alkyl or alkenyl group which has 1 to 10 C atoms and is optionally substituted by halogen, or halogen,

at temperatures as a rule between −30°C and 150°C, preferably between 0 and 50°C, if appropriate in an inert organic solvent.

8. Use of compounds according to Claims 1 to 6 as activators in free radical polymerisation of monomers containing olefinic groups.

9. Use according to Claim 8, characterised in that 0.1 to 5% by weight of activator, based on the monomer(s), is used.

10. Polymerisable composition containing an olefinically unsaturated monomer, a tertiary amine, as the activator, and, if appropriate, organic and/or inorganic fillers and/or other auxiliaries and additives which are known per se, characterised in that the activator is a compound according to Claims 1 to 6.

**Revendications**

1. Composé de formule générale

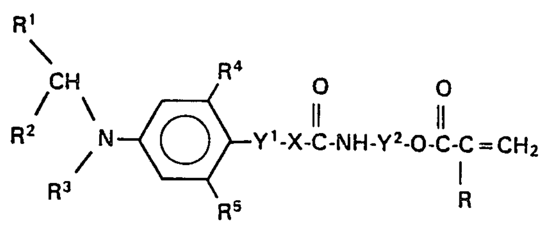

(I)

dans laquelle

R désigne l'hydrogène ou un groupe méthyle,

$Y^1$ est un reste alkylène éventuellement ramifié ayant 1 à 6 atomes de carbone, de préférence 1 à 3 atomes de carbone et

$Y^2$ représente un reste alkylène éventuellement ramifié ayant 2 à 8 atomes de carbone, de préférence 2 à 5 atomes de carbone,

X est l'oxygène ou un groupe -NH-,

$R^1$ et $R^2$ sont identiques ou différents et représentent l'hydrogène, un groupe alkyle, alcényle, cycloalkyle, cycloalcényle, aryle, aralkyle ou alkaryle ayant 1 à 11 atomes de carbone, éventuellement substitué par un ou plusieurs (de préférence un seul) groupes hydroxy, amino, époxy, uréthanne, urée, ester ou éther, ou bien

$R^1$ et $R^2$ forment conjointement un noyau de 3 à 6 chaînons qui comprend éventuellement de l'azote, de l'oxygène ou du soufre comme hétéroatomes,

$R^3$ a la définition de $R^1$ ou représente un groupe

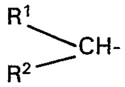

ou bien

$R^2$ et $R^3$ forment en association avec le groupe
| |
-CH-N- un noyau pentagonal ou hexagonal qui comprend éventuellement de l'azote comme autre hétéroatome, et

$R^4$ et $R^5$ sont identiques ou différents et représentent de l'hydrogène, un groupe alkyle ou alcényle éventuellement substitué par un halogène, ayant 1 à 10 atomes de carbone, ou un halogène.

2. Composés suivant la revendication 1, caractérisés en ce que le reste $R^1$ représente l'hydrogène.

3. Composés suivant la revendication 1 ou 2, caractérisés en ce que $R^2$ représente l'hydrogène ou le groupe méthyle.

4. Composés suivant les revendications 1 à 3, caractérisés en ce que $R^3$ est un groupe méthyle ou éthyle.

5. Composés suivant les revendications 1 à 4, caractérisés en ce que $R^4$ et $R^5$ représentent l'hydrogène ou un groupe méthyle.

6. Composés suivant la revendication 5, caractérisés en ce que $R^4$ et $R^5$ représentent tant l'un que l'autre, l'hydrogène.

7. Procédé de production de composés suivant les revendications 1 à 6, caractérisé en ce qu'il consiste à faire réagir à des températures en général comprises entre -30 et 150°C, de préférence entre 0 et 50°C, éventuellement dans un solvant organique inerte, des amines tertiaires de formule générale

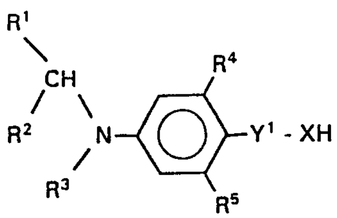

(II)

avec des isocyanates de formule générale

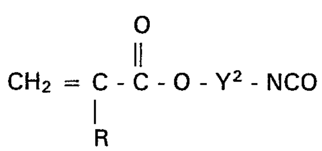

(III)

formules dans lesquelles

R représente l'hydrogène ou un groupe méthyle,

$Y^1$ est un reste alkylène éventuellement ramifié ayant 1 à 6 atomes de carbone, de préférence 1 à 3 atomes de carbone et

$Y^2$ est un reste alkylène éventuellement ramifié ayant 2 à 8 atomes de carbone, de préférence 2 à 5 atomes de carbone,

X désigne l'oxygène ou un groupe -NH-

$R^1$ et $R^2$ sont identiques ou différents et représentent l'hydrogène, un groupe alkyle, alcényle, cycloalkyle, cycloalcényle, aryle, aralkyle ou alkaryle ayant 1 à 11 atomes de carbone, éventuellement substitué par un ou plusieurs (de préférence un seul) groupes hydroxy, amino, époxy, uréthanne, urée, ester ou éther, ou bien

$R^1$ et $R^2$ formant ensemble un noyau de 3 à 6 chaînons qui contient éventuellement de l'azote, de l'oxygène ou du soufre comme hétéroatomes,

$R^3$ a la définition de $R^1$ ou représente un groupe

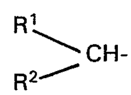

ou bien

$R^2$ et $R^3$ forment conjointement avec le groupe
| |
-CH-N- un noyau pentagonal ou hexagonal qui contient éventuellement de l'oxygène comme autre hétéroatome, et

$R^4$ et $R^5$ sont identiques ou différents et représentent de l'hydrogène, un groupe alkyle ou alcényle éventuellement substitué par un halogène, ayant 1 à 10 atomes de carbone, ou un halogène.

8. Utilisation de composés suivant les revendications 1 à 6 comme activateurs dans la polymérisation radicalaire de monomères portant des groupes oléfiniques.

9. Utilisation suivant la revendication 8, caractérisé en ce qu'on met en jeu 0,1 à 5% en poids d'activateur, par rapport au monomère ou aux monomères.

10. Mélange polymérisable, contenant un monomère à insaturation oléfinique, une amine tertiaire comme activateur ainsi que, le cas échéant, des charges organiques et/ou inorganiques et/ou d'autres adjuvants et additifs connus, caractérisé en ce que l'activateur est un composé suivant les revendications 1 à 6.